# EUROPEAN PATENT SPECIFICATION

(11) **EP 4 006 155 B1**
(45) Date of publication and mention of the grant of the patent: **17.09.2025**
(21) Application number: 20846919.7
(22) Date of filing: 22.07.2020
(51) Int. Cl.: C12Q 1/6848, C12Q 1/6883

(54) **METHOD FOR DETECTING OR QUANTIFYING SMN1 GENE**
VERFAHREN ZUR DETEKTION ODER QUANTIFIZIERUNG VON SMN1-GEN
PROCEDE DE DETECTION OU DE QUANTIFICATION D'UN GENE SMN1

(30) Priority: 26.07.2019 JP 2019137633; 25.03.2020 JP 2020054242
(43) Date of publication of application: 01.06.2022
(73) Proprietor: Sekisui Medical Co., Ltd., Tokyo 103-0027 (JP)
(72) Inventor: EBINUMA Hiroyuki, Tokyo 103-0027 (JP); SUZUKI Ikumi, Tokyo 103-0027 (JP)
(74) Representative: Hoffmann Eitle
(86) International application number: PCT/JP2020/028374
(87) International publication number: WO 2021/020261

(56) References cited:
- WO-A1-2018/117986
- WO-A1-2019/074004
- WO-A1-2019/074004
- WO-A1-2020/219751
- WO-A2-2014/210199
- CN-A- 103 555 835
- CN-A- 103 555 835
- JP-A- 2016 526 390
- JP-A- 2017 533 722
- SCHOLL RAPHAEL ET AL: "Spinal Muscular Atrophy: Position and Functional Importance of the Branch Site Preceding SMN Exon 7", RNA BIOLOGY, vol. 4, no. 1, 27 January 2007 (2007-01-27), pages 34 - 37, XP093057616, ISSN: 1547-6286, DOI: 10.4161/rna.4.1.4534
- AR ROCHMAH MAWADDAH ET AL: "Genetic screening of spinal muscular atrophy using a real-time modified COP-PCR technique with dried blood-spot DNA", BRAIN & DEVELOPMENT, AMSTERDAM, NL, vol. 39, no. 9, 15 May 2017 (2017-05-15), pages 774 - 782, XP085157570, ISSN: 0387-7604, DOI: 10.1016/J.BRAINDEV.2017.04.015
- M. HAYASHIDA: "Genotyping of polymorphisms in alcohol and aldehyde dehydrogenase genes by direct application of PCR-RFLP on dried blood without DNA extraction", ANALYTICAL SCIENCES, vol. 26, 1 January 2010 (2010-01-01), pages 503 - 505, XP055461972
- FELDKÖTTER MARKUS, SCHWARZER VERENA, WIRTH RADU, WIENKER THOMAS F., WIRTH BRUNHILDE: "Quantitative analyses of SMN1 and SMN2 based on real-time lightcycler PCR: Fast and highly reliable carrier testing and prediction of severity of spinal muscular atrophy", THE AMERICAN JOURNAL OF HUMAN GENETICS, vol. 70, no. 2, February 2002 (2002-02-01), pages 358 - 368, XP055788100

## Description

### TECHNICAL FIELD

The present invention relates to a method for specifically detecting and quantifying the *SMN1* gene contained in a punched piece by direct real-time PCR using a punched piece of a certain size obtained from a dried blood spot in a filter paper and to the use of a primer for the method.

### BACKGROUND ART

Spinal muscular atrophy (SMA) is a lower motor neuron disease characterized by muscular atrophy and progressive muscle weakness due to degeneration of the anterior horn cells in the spinal cord and is designated to the incurable disease. There are four types of SMA depending on the age of symptom onset and the degree. In particular, in type I with the onset within about six months after birth, the diagnosis tends to be delayed also because of the onset in the neonatal period, and the symptoms tend to become severe. Ninety percent or more of the SMA type I patients die before 2 years of age or require a ventilator for the whole life. The prevalence of type I is around one in 20000 births, which is high, and it is known that therapeutic effects are expected more as the treatment is started earlier. Thus, request for newborn screening test is next to that for primary immunodeficiencies. It is known that 95% of the SMA type I patients are caused by homozygous deletion of survival of motor neuron 1 (*SMN1*) gene, which plays a role of maintaining the functions of the motor neuron, and thus the gene to be screened is the *SMN1* gene. However, in the *SMN2* gene, which compensates the gene, the 6th nucleotide in exon 7 is T instead of C in the *SMN1* gene, and thus the sequence causes skipping of exon 7 during the splicing. Therefore, the single nucleotide polymorphism is preferably used as the specific target. In a method for specifically measuring a single nucleotide polymorphism, an attempt is often made to secure the specificity by designing allele-specific primers, but it is often difficult to secure complete specificity depending on the nucleotide sequence around the single nucleotide polymorphism as the subject. When a system for measuring the *SMN1* gene for newborn screening application is developed, the analyte to be measured is a paper piece cut out of a dried blood spot in a filter paper, and thus considerable ingenuity is required for the elution of blood from the paper piece, elution of an interfering substance and the like.

In Non-Patent Document 1, the specificity to the *SMN1* gene is secured by designing a fluorescently labeled LNA (Locked Nucleic Acid) probe to the 110th position of the two single nucleotide polymorphisms in intron 7 of the *SMN2* gene instead of one in exon 7 and thus specifically inhibiting the amplification of the *SMN2* gene.

In Non-Patent Document 2, a measurement method using the melting curve assay of the amplification product of PCR reaction which is performed using primers designed to flank the sixth nucleotide of exon 7 is reported. The method is not suitable for screening test because not only an amplification product of the *SMN1* gene but also an amplification product of the *SMN2* gene is generated and because the melting curves thus overlap each other and are difficult to assess.

In the method reported in Non-Patent Document 3, a part of the reaction solution of first PCR reaction performed using primers designed to flank the sixth nucleotide of exon 7 is used for second PCR reaction using short primers in which complementary nucleotides to the *SMN1* gene and the *SMN2* gene are located in the middle, and the amplification product is analyzed by electrophoresis. The method is a method which uses the principles of so-called nested-PCR, which is a method used when the specificity of the primers in the first reaction is insufficient. A major defect of the method is an increase in the risk of contamination to the environment caused by the scattering of the amplification product due to opening of the cover after the first PCR reaction. Moreover, the amplification product is detected as a band of the amplification product through electrophoresis, and thus the method is not suitable for screening test also in view of the measurement efficiency.

Non-Patent Document 4 reports a method for quantifying the *SMN1* gene by real-time PCR reaction which is performed using primers designed to flank the sixth position of exon 7 and a fluorescent dye-modified probe which is complementary to the sixth position of exon 7. In the method, because an amplification product derived from the *SMN2* gene is also generated at the same time, there is a concern that the specificity of the probe cannot be secured when the CNV (Copy Number Variation) of the *SMN2* gene is high.

In Non-Patent Documents 5 and 6, although primers similar to those of the invention are designed and used, purified DNA is used as an analyte in both documents, and the methods are not suitable for screening test also in view of the measurement efficiency.

WO2019074004A1 relates to a SNP detection method to detect SMN1

### CITATION LIST

### NON PATENT LITERATURE

[Non-Patent Document 1] Clinical Chemistry, 61:2, 412-419, 2015
[Non-Patent Document 2] Clinical Chemistry, 58:6, 1033-1039, 2012
[Non-Patent Document 3] Kobe J.Med.Sci., 63:2, E37-40, 2017
[Non-Patent Document 4] Genet Med, 8:7, 428-437, 2006
[Non-Patent Document 5] Neurogenetics 8, 271-278, 2007
[Non-Patent Document 6] Am.J.Hum.Genet. 70, 358-368, 2002

### SUMMARY OF INVENTION

### TECHNICAL PROBLEM

An object of the invention is to provide a primer for detecting and quantifying homozygous deletion of the *SMN1* gene, the deletion of which causes SMA, using a dried blood spot in a filter paper and a method related to specific detection/quantification of the *SMN1* gene using the primer.

### SOLUTION TO PROBLEM

Present invention is defined by the appended claims.

Although SMA has been considered as a mortal disease so far, a therapeutic agent has been developed recently, and treatment has been started in clinical sites. Thus, improvement of QOL is expected. In particular, symptoms of type I tend to become severe, and the treatment is desirably started at the earliest possible stage. In view of the request, a versatile and rapid detection/quantification system which can be applied to newborn screening and which is highly specific to the *SMN1* gene is desired.

By designing and using a primer which is highly specific to the *SMN1* gene and with which the reactivity to the *SMN2* gene is extremely lower than the reactivity to the *SMN1* gene in a method for detecting the *SMN1* gene by direct real-time PCR using a dried blood spot in a filter paper, a method for detecting and quantifying the *SMN1* gene which can determine whether the *SMN1* gene is deleted or not has been established. The invention has been thus completed and it is defined in the appended claims.

### ADVANTAGEOUS EFFECTS OF INVENTION

According to the disclosure, the provision of a primer which amplifies a specific region of the *SMN1* gene in real-time PCR using a dried blood spot in a filter paper and which does not easily amplify the *SMN2* gene and has low reactivity has been enabled. Moreover, using the primer, the provision of a method for specifically detecting or quantifying the *SMN1* gene in a dried blood spot in a filter paper by real-time PCR has been enabled.

Accordingly, a rapid detection/quantification system for the *SMN1* gene that can be applied to newborn screening in which the symptoms tend to become severe and in which the treatment is desirably started at an early stage can be provided.

### BRIEF DESCRIPTION OF DRAWINGS

[Fig. 1] The amplification reaction curves of the PCR reagent containing SMN1 standards for a calibration curve are shown.
[Fig. 2] The parts of the amplification reaction curves of the PCR reagent for SMN2 cross test at 25 to 40 cycles are shown.

### DESCRIPTION OF EMBODIMENTS

### (SMN1 Gene-Specific Primer)

The *SMN1* gene-specific primer of the invention is a primer designed in a manner that the primer specifically amplifies the *SMN1* gene and does not easily amplify the *SMN2* gene. That is, the primer is a primer designed in a manner that the reactivity to the *SMN2* gene is less than 1% of that to the *SMN1* gene. The reactivity is determined as follows. Assuming that the reactivity is 100% when the Cq values are the same in the case where the copy numbers of the *SMN1* gene and the *SMN2* gene per reaction are the same, the reactivity is 10% when the Cq values are the same in the case where the copy number of the *SMN2* gene per reaction is 10 times higher than that of the *SMN1* gene, and the reactivity is 1% when the Cq values are the same in the case where the copy number is 100 times higher. Here, when the Cq value of the *SMN1* gene is smaller, the reactivity is determined to be less than 1%. In the Examples described below, of the combinations of the conditions for the primers and the amplification elongation temperature, when the ΔCq value which was obtained by subtracting the Cq value of the *SMN2* gene 10,000 copy/reaction from the Cq value of the *SMN1* gene 100 copy/reaction was less than -0.1, the reactivity was determined to be less than 1%.

It has been also found that, when PCR reaction is performed using the primer of the invention, the reactivity to the *SMN2* gene further weakens in the case where a dried blood spot in a filter paper is used as an analyte.

The forward primer which is specific to the *SMN1* gene of the invention may

The reverse primer which is specific to the *SMN1* gene of the invention may be a primer having any of the nucleotide sequences of SEQ ID NOs: 16 to 21. The reverse primers are designed to contain the complementary sequence to the sixth position of exon 7 of the *SMN1* gene at the 3' end of the reverse primers and have a length of 20 to 25 bases. The successive bases are preferably identical to the corresponding complementary strand of the *SMN1* gene, but a primer having a difference by one or some bases which do not hinder the amplification of the target nucleic acid is also included.

As the primer of the invention, either of the forward primer which is specific to the *SMN1* gene and the reverse primer which is specific to the *SMN1* gene may be used, and the reverse primer and the forward primer used as a set are desirably 20 bp or more apart in the case of real-time PCR considering the necessity for designing a fluorescently labeled probe in the region between the primers.

The reverse primer used with the forward primer which is specific to the *SMN1* gene of the invention as a set may be the reverse primer which is specific to the *SMN1* gene of the invention, or a primer containing a complementary sequence to a be specifically a primer having any of the nucleotide sequences of SEQ ID NO 9. The forward primers are designed to contain C at the sixth position of exon 7 of the *SMN1* gene at the 3' end. The forward primers have a length of 22 to 30 bases in succession from C at the sixth position toward the 5' end. The successive bases are preferably identical to the corresponding nucleotide sequence in the *SMV1* gene, but a primer having a difference by one or some bases which do not hinder the amplification of the target nucleic acid is also included . nucleotide sequence which is identical to a part of the exon 7 or intron 7 region as long as the forward and reverse primers are 20 bp or more apart.

The forward primer used with the reverse primer which is specific to the *SMN1* gene of the invention as a set may be the forward primer which is specific to the *SMN1* gene of the invention or a primer containing a nucleotide sequence which is identical to a part of the intron 6 region.

When PCR is performed using the forward primer which is specific to the *SAM1* gene or the reverse primer which is specific to the *SMN1* gene of the invention, nucleic acid containing C at the sixth position of exon 7 of the *SMN1* gene is amplified. On the other hand, because the sixth position of exon 7 of the corresponding nucleic acid of the *SMN2* gene is T, which is not recognized by the 3' end of the forward primer which is specific to the *SMN1* gene or the reverse primer which is specific to the *SMN1* gene of the invention, the amplification reaction by the polymerase is not caused easily. Moreover, when a dried blood spot in a filter paper is used as an analyte, the amplification reaction of the *SMN2* gene becomes further difficult, and specific detection of the *SMN1* gene is thus enabled.

In this manner, when a dried blood spot in a filter paper is used as an analyte for PCR using at least one kind of the forward primer which is specific to the *SMN1* gene and the reverse primer which is specific to the *SMN1* gene of the invention, the effects can be exerted more. That is, because the region in exon 7 of the *SUN1* gene can be amplified specifically and because the region in the *SMN2* gene is hardly amplified, the *SMN1* gene can be detected or quantified specifically. The amplified nucleic acid can be qualitatively or quantitatively analyzed by electrophoresis, real-time PCR or the like.

It has been demonstrated in the Examples described below that the reactivity to the *SMN2* gene is less than 1% of that to the *SMN1* gene when PCR is performed using the forward primer which is specific to the *SAM1* gene or the reverse primer which is specific to the *SMN1* gene of the invention.

### (PCR/Real-Time PCR)

The real-time PCR method generally means a method for sequentially monitoring the process of generation of an amplification product in PCR. In the invention, detection by real-time PCR means detection of an amplified target nucleic acid in the *SMN1* gene (also simply referred to as an amplification product below) by optical means after the PCR reaction. Here, the amplification product may be detected, for example, at the time of completion of the PCR reaction or after the completion or may be detected simultaneously during the PCR reaction step. In the case of simultaneous detection, the amplification product can be detected, for example, sequentially. The sequential detection (monitoring) may be, for example, continuous or noncontinuous (intermittent) detection.

The temperature changes of the steps in the PCR reaction may be automatically controlled using, for example, a thermal cycler or the like. The amplification product generated by the PCR reaction can be detected, for example, by detecting the fluorescence intensity generated from the amplification product. The fluorescence detection is not particularly restricted but is the conventionally known intercalator method, the TaqMan (registered trademark) probe method, the hybridization method, the cycling probe method or the like.

The fluorescence intensity can be detected, for example, with a fluorometer. Moreover, in general, an apparatus which has both a PCR reaction unit (for example, a thermal cycler) and an optical unit (for example, a fluorometer) is used. Specific examples include commercial SMartCycler (product name, manufactured by Takara Bio Inc.), LightCycler (product name, manufactured by Roche Diagnostics), ABI PRISM7000 (product name, manufactured by Applied Biosystems) and the like.

### (Quantification Method of Target Nucleic Acid)

The method for quantifying the *SMN1* gene of the invention is a method of generating an amplification product complementary to a target nucleic acid in the *SMN1* gene in a sample, detecting the amplification product by optical means and quantifying the amplification product. By counting the PCR cycle number at which the amount of the amplification product has reached a certain amount, the target nucleic acid contained in the sample can be quantified in the method. Moreover, as in the Examples described below, the quantification can be conducted by calculating the copy number of 1 µL of whole blood in the sample from a calibration curve of the Cq values calculated from the standards.

### (PCR Reagent)

The PCR reagent used in the invention contains at least a set of primers, a polymerase, dNTPs (deoxynucleoside triphosphates), an intercalator or a fluorescently labeled probe. The reagent also typically contains a buffer. The buffer is not particularly limited as long as the buffer has the function of inhibiting the activities of substances which inhibit the DNA amplification reaction, such as positively charged substances (certain kinds of protein and the like) and negatively charged substances (certain kinds of saccharide, dyes and the like), in the body fluid of a living thing. Examples of commercial buffers include Ampdirect, Ampdirect plus (both manufactured by Shimadzu Corporation) and the like. The amount of the PCR reagent used in the invention added to a reaction tube is 20 to 50 µL.

### (Sample/Dried Blood Spot in a Filter Paper)

The sample used in the invention is preferably blood in a filter paper that is obtained by blotting blood on the filter paper and then drying the blood, which is called a dried blood spot in a filter paper. The dried blood spot in a filter paper is taken out using a punching device as a paper piece having the shape of the punching hole (punched piece). Examples of the dried blood spot in a filter paper used include blood on a filter paper collected from the heel of a newborn during newborn mass screening or the like, blood on a filter paper collected during simultaneous optional screening and the like, but the dried blood spot in a filter paper is not limited to the examples. Regarding the size of the punched piece that is cut out from the dried blood spot in a filter paper, a punched piece having a certain size is desirable because the amount of the contained blood should be in an appropriated range. The punched piece having a certain size is desirably a circular punched piece with a diameter of 1.2 to 2.0 mm, further desirably a circular punched piece with a diameter of 1.5 mm to 1.8 mm. The whole blood amount contained in the punched piece based on the total amount of the PCR reaction solution is desirably 0.95 v/v% to 6.6 v/v%, further desirably 1.4 v/v% to 5.2 v/v%. Here, the whole blood amount is indicated as the proportion which is obtained by adding a circular punched piece with a diameter of 1.2 to 2.0 mm or a circular punched piece with a diameter of 1.5 mm to 1.8 mm to 20 to 50 µL of a PCR reaction solution and dissolving the blood and which is calculated based on the actual value (when 30 µL of whole blood was added to a filter paper before cutting out a punched piece and dried, the diameter of the created circular blood portion was 9.5 mm).

### (PCR Reaction Tube)

The tube used in the invention is a tube for PCR reaction and has a structure into which a punched piece of the filter paper with blood can be smoothly inserted and which can be sealed with a cap. In a suitable shape, the opening at the upper part of the tube is a circle (for example, an inside diameter of around 2.5 mm to 10 mm), and the bottom is narrower than the opening at the upper part. An inverted cone shape or the like is preferable.

The volume of the tube may be a volume to which the PCR reaction reagent can be added and which can sufficiently hold the reaction solution even with the temperature changes during the PCR reaction, and the tube preferably has a volume of 0.1 mL to 0.3 mL, further preferably a volume of 0.15 mL to 0.25 mL, most preferably a volume of 0.2 mL.

The PCR reaction tube used in the invention is preferably a 96-well plate for PCR reaction in which 96 tubes are connected or one in an eight-tube strip in which eight tubes are connected, and as commercial products, those sold with a name 96-well PCR plate, PCR eight-tube strip or the like can be used. Commercial products include LightCycler (registered trademark) 480 Multiwell Plate 96 (Roche, Inc.), 96-Well PCR Plate, Flat Top, Low Profile, Natural, Polypropylene, UltraFlux (Scientific Specialties, Inc.), Eppendorf PCR plate 96, skirtless, 150 µL, colorless (Eppendorf), PCR plate 96-well simplate 0.1 mL natural (BM Equipment Co., Ltd.),

### LightCycler 8-Tube Strips (Roche, Inc.) and the like.

The material of such a reaction tube desirably causes little contamination of the reaction solution and is stiff so that the shape does not change even with the changes in the internal pressure due to the temperature changes during the PCR reaction, and the tube is made of polypropylene, for example.

### EXAMPLES

The present invention is explained in detail below by Examples, but the invention is not limited to the Examples below.

The present Examples are examples which compared the reactivities of the designed primers to the *SMN1* gene and *SMN2* gene sequences.

### [Example 1] SMN1 Gene Quantification Systems Using SMN1 Gene-Specific Forward Primers

### (a) Test Materials

### (a-1) SMN1 Gene Amplification Forward Primers

Forward primers for the *SMN1* gene amplification (Table 1; SEQ ID NOs: 1 to 9) and a common reverse primer (Table 1; SEQ ID NO: 10) were synthesized by Sigma-Aldrich Co. LLC and used.

**[Table 1]**

| Table 1: *SMN1* Gene-Specific Forward Primers of the Present Invention and Common Reverse Primer | | | | |
|---|---|---|---|---|
| SEQ ID NO | Primer Name | Nucleotide Sequence (5'→3') | bp | Tm |
| 1 | SMN1_ASP30 | TAACTTCCTTTATTTTCCTTACAGGGTTTC | 30 | 65.6 |
| 2 | SMN1_ASP29 | AACTTCCTTTATTTTCCTTACAGGGTTTC | 29 | 65.8 |
| 3 | SMN1_ASP28 | ACTTCCTTTATTTTCCTTACAGGGTTTC | 28 | 65.1 |
| 4 | SMN1_ASP27 | CTTCCTTTATTTTCCTTACAGGGTTTC | 27 | 64.6 |
| 5 | SMN1_ASP26 | TTCCTTTATTTTCCTTACAGGGTTTC | 26 | 64.0 |
| 6 | SMN1_ASP25 | TCCTTTATTTTCCTTACAGGGTTTC | 25 | 63.1 |
| 7 | SMN1_ASP24 | CCTTTATTTTCCTTACAGGGTTTC | 24 | 61.7 |
| 8 | SMN1_ASP23 | CTTTATTTTCCTTACAGGGTTTC | 23 | 58.7 |
| 9 | SMN1_ASP22 | TTTATTTTCCTTACAGGGTTTC | 22 | 57.8 |
| 10 | SMN_RP(1) | CACTTTCATAATGCTGGCAGACTTAC | 26 | 65.5 |

### (a-2) Plasmids

As templates of the PCR reaction, plasmids having a partial sequence shown below which included exon 7 of the *SMN1* gene or the *SMN2* gene and which was incorporated in a vector were synthesized by Eurofins Scientific SE and used. The underlined part of SEQ ID NO: 11 below shows the sequence of exon 7 (54 bp) of the *SMN1* gene, where the upstream is a partial sequence of intron 6, and the downstream shows a partial sequence of intron 7. The underlined part of SEQ ID NO: 12 shows the sequence of exon 7 (54 bp) of the *SMN2* gene, where the upstream is a partial sequence of intron 6, and the downstream shows a partial sequence of intron 7.

The sixth position of exon 7 of the *SAM1* gene is C, while the sixth position of exon 7 of the *SMN2* gene is T.

Partial Sequence of *SMN1* Gene Including Exon 7 (SEQ ID NO: 11) 
CAACTTAATTTCTGATCATATTTTGTTGAATAAAATAAGTAAAATGTCTTGTG
AAACAAAATGCTTTTTAACATCCATATAAAGCTATCTATATATAGCTATCTATG
TCTATATAGCTATTTTTTTTAACTTCCTTTATTTTCCTTACAGGGTTTCAGACA
AAATCAAAAAGAAGGAAGGTGCTCACATTCCTTAAATTAAGGAGTAAGTCT
GCCAGCATTATGAAAGTGAATCTTACTTTTGTAAAACTTTATGGTTTGTGGAA
AACAAATGTTTTTGAACATTTAAAAAGTTCAGATGTTAAAAAGTTGAAAGGT
TAATGTAAAACAATCAATATTAAAGAATTTTGATGCCAAAACTATTAGATAAA
AGGTTAATCTACATCCCTACTAGAATTCTC
Partial Sequence of *SMN2* Gene Including Exon 7 (SEQ ID NO: 12)

CAACTTAATTTCTGATCATATTTTGTTGAATAAAATAAGTAAAATGTCTTGTG

AAACAAAATGCTTTTTAACATCCATATAAAGCTATCTATATATAGCTATCTATAT

CTATATAGCTATTTTTTTTAACTTCCTTTATTTTCCTTACAGGGTTTTAGACAA
AATCAAAAAGAAGGAAGGTGCTCACATTCCTTAAATTAAGGAGTAAGTCTG
CCAGCATTATGAAAGTGAATCTTACTTTTGTAAAACTTTATGGTTTGTGGAAA
ACAAATGTTTTTGAACATTTAAAAAGTTCAGATGTTAGAAAGTTGAAAGGTT
AATGTAAAACAATCAATATTAAAGAATTTTGATGCCAAAACTATTAGATAAAA
GGTTAATCTACATCCCTACTAGAATTCTC

### (b) PCR Reagent

The composition of the PCR reagent is shown below.
1× (final concentration) PCR buffer (Ampdirect Plus; Shimadzu Corporation)
0.2 µM (final concentration) *SMN1* gene forward primer (SEQ ID NOs: 1 to 9)
0.2 µM (final concentration) common reverse primer (SEQ ID NO: 10)
0.025 U/µL (final concentration) BIOTAQ HSDNA polymerase
0.5× (final concentration) EvaGreen

### (c) Plasmid Dilution Series

*SMN1* gene plasmid (final concentration) 100,000 copy/reaction, 10,000 copy/reaction, 1,000 copy/reaction, 100 copy/reaction
*SMN2* gene plasmid (final concentration) 100,000 copy/reaction, 10,000 copy/reaction

### (d) Conditions for PCR Reaction

(i) 95°C: 15 minutes
(ii) 95°C: 15 seconds, 61 to 65°C: 80 seconds (45 cycles)
(iii) 37°C: 5 minutes

### (e) Real-Time PCR Measurement Using Intercalator

### (e-1) Measurement Method

The measurement was made by the following procedures.
(i) The PCR reagent and the plasmids were mixed at the final concentrations above, and PCR tubes were prepared by dispensing 40 µL thereof.
(ii) Using a thermal cycler (CFX96; Bio-Rad Laboratories, Inc.), real-time PCR measurement was made, and the Cq values of the respective plasmid concentrations were calculated.

### (e-2) Measurement Results

The results are shown in Table 2. Of the combinations of the conditions for the primers and the amplification elongation temperature, there were 11 combinations in which the reactivity to the *SMN2* gene was less than 1% of that to the *SMN1* gene, that is, there were 11 combinations that satisfied the ΔCq value of less than -0.1, where the ΔCq value was obtained by subtracting the Cq value of the *SMN2* gene 10,000 copy/ reaction from the Cq value of the *SMN1* gene 100 copy/ reaction. The forward primers of SEQ ID NOs: 1 to 9 were applicable.

**[Table 2]**

| Table 2: Measurement Results of Real-Time PCR Using SMN1 Gene-Specific Forward Primers of The Present Invention | | | | | | | | | | | | | | |
|---|---|---|---|---|---|---|---|---|---|---|---|---|---|---|
| Amplification Elongation Temperature | 61°C | | | 63°C | | | | | | 65°C | | | | |
| Forward Primer | ASP25 | ASP24 | ASP23 | ASP28 | ASP27 | ASP26 | ASP25 | ASP24 | ASP23 | ASP30 | ASP29 | ASP28 | ASP27 | ASP26 |
| SMN1 1x10⁵ copy | 23.7 | 23.9 | 24.1 | 24.0 | 24.1 | 24.2 | 24.2 | 24.2 | 25.5 | 24.0 | 24.0 | 24.1 | 24.2 | 25.1 |
| SMN1 1x10⁴ copy | 27.2 | 27.3 | 27.5 | 27.3 | 27.3 | 27.4 | 27.4 | 27.6 | 28.8 | 27.4 | 27.3 | 27.3 | 27.4 | 28.3 |
| SMN1 1x10³ copy | 30.5 | 30.6 | 30.7 | 30.7 | 30.7 | 30.7 | 30.7 | 31.0 | 32.1 | 31.0 | 30.6 | 30.6 | 30.6 | 31.6 |
| *SMN1 1×10² copy* 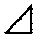 | ***34.2*** | ***33.7*** | ***34.3*** | ***33.9*** | ***34.3*** | ***34.0*** | ***34.2*** | ***34.0*** | ***35.4*** | ***34.0*** | ***33.6*** | ***34.2*** | ***33.9*** | ***34.9*** |
| SMN2 1x10⁵copy | 30.0 | 30.6 | 32.8 | 28.9 | 30.3 | 32.5 | 32.6 | 32.8 | 34.3 | 31.7 | 31.8 | 32.5 | 33.0 | 33.7 |
| *SMN2 1×10⁴ copy* | ***33.3*** | ***33.7*** | ***36.0*** | ***32.2*** | ***33.6*** | ***35.5*** | ***35.9*** | ***36.7*** | ***37.9*** | ***34.8*** | ***35.2*** | ***35.9*** | ***35.8*** | ***37.3*** |
| Cq Value 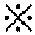 | 0.8 | 0.0 | ***-1.7*** | 1.7 | 0.7 | ***-1.5*** | ***-1.7*** | ***-2.7*** | ***-2.5*** | ***-0.8*** | ***-1.6*** | ***-1.7*** | ***-1.9*** | ***-2.4*** |

| | | | | | | | | | | | | | | |
|---|---|---|---|---|---|---|---|---|---|---|---|---|---|---|
| 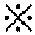 SMN1 1×10²copy Cq Value - SMN2 1×10⁴copy Cq Value | | | | | | | | | | | | | | |

### [Example 2] SMN1 Gene Quantification Systems Using SMN1 Gene-Specific Reverse Primers

### (f) Test Materials

### (f-1) SMN1 Gene Amplification Reverse Primers

Reverse primers for the *SMN1* gene amplification (Table 3; SEQ ID NOs: 14 to 22) and a common forward primer (Table 3; SEQ ID NO: 13) were synthesized by Sigma-Aldrich Co. LLC and used.

**[Table 3]**

| Table 3: *SMN* Gene-Specific Reverse Primers of the Present Invention and Common Forward Primer | | | | |
|---|---|---|---|---|
| SEQ ID NO | Primer Name | Nucleotide Sequence (5'→3') | bp | Tm |
| 13 | SMN_FP(1) | AGTAAAATGTCTTGTGAAACAAAATGCT | 28 | 64.5 |
| 14 | SMN1_RASP27 | CACCTTCCTTLIIIIIGATTTTGTCTG | 27 | 67.3 |
| 15 | SMN1_RASP26 | ACCTTCCTTCTTTTTGATTTTGTCTG | 26 | 65.2 |
| 16 | SMN1_RASP25 | CCTTCCTTCTTTTTGATTTTGTCTG | 25 | 64.7 |
| 17 | SMN1_RASP24 | CTTCCTTCTTTTTGATTTTGTCTG | 24 | 61.8 |
| 18 | SMN1_RASP23 | TTCCTTCTTTTTGATTTTGTCTG | 23 | 61.0 |
| 19 | SMN1_RASP22 | TCCTTCTTTTTGATTTTGTCTG | 22 | 59.8 |
| 20 | SMN1_RASP21 | CCTTCTTTTTGATTTTGTCTG | 21 | 58.0 |
| 21 | SMN1_RASP20 | CTTCTTTTTGATTTTGTCTG | 20 | 54.0 |
| 22 | SMN1_RASP19 | TTCTTTTTGATTTTGTCTG | 19 | 52.7 |

### (f-2) Plasmids

The same plasmids as those in (a-2) above were used.

### (g) PCR Reagent

The composition of the PCR reagent is shown below.
1× (final concentration) PCR buffer (AMpdirect Plus; Shimadzu Corporation)
0.2 µM (final concentration) common forward primer (SEQ ID NO: 13)
0.2 µM (final concentration) *SMN1* gene reverse primer (SEQ ID NOs: 14 to 22)
0.025 U/µL (final concentration) BIOTAQ HSDNA polymerase
0.5× (final concentration) EvaGreen

### (h) Plasmid Dilution Series

The same plasmid dilution series as those in (c) above were used.

### (i) Conditions for PCR Reaction

The reaction was performed under the same conditions as those in (d) above.

### (j) Real-Time PCR Measurement Using Intercalator

### (j-1) Measurement Method

The measurement was made by the following procedures.
(i) The PCR reagent and the plasmids were mixed at the final concentrations above, and PCR tubes were prepared by dispensing 40 µL thereof.
(ii) Using a thermal cycler (CFX96; Bio-Rad Laboratories, Inc.), real-time PCR measurement was made, and the Cq values of the respective plasmid concentrations were calculated.

### (j-2) Measurement Results

The results are shown in Table 4. Of the combinations of the conditions for the primers and the amplification elongation temperature, there were 8 combinations in which the reactivity to the *SMN2* gene was less than 1% of that to the *SMN1* gene, that is, there were 8 combinations that satisfied the ΔCq value of less than -0.1, where the ΔCq value was obtained by subtracting the Cq value of the *SMN2* gene 10,000 copy/reaction from the Cq value of the *SMN1* gene 100 copy/reaction. The reverse primers of SEQ ID NOs: 16 to 21 were applicable.

**[Table 4]**

| Table 4: Measurement Results of Real-Time PCR Using Reverse Primers of The Present invention | | | | | | | | | | | | |
|---|---|---|---|---|---|---|---|---|---|---|---|---|
| Amplification Elongation Temperature | 61°C | | | | 63°C | | | | 65°C | | | |
| Reverse Primer | RASP22 | RASP21 | RASP20 | RASP19 | RASP24 | RASP23 | RASP22 | RASP21 | RASP27 | RASP26 | RASP25 | RASP24 |
| SMN1 1x10⁵copy | 23.4 | 23.5 | 26.7 | 0.0 | 23.0 | 23.8 | 24.2 | 25.8 | 23.4 | 23.4 | 23.6 | 24.8 |
| SMN1 1x10⁴copy | 26.8 | 27.0 | 30.4 | 0.0 | 26.3 | 27.2 | 27.8 | 29.2 | 26.8 | 26.8 | 27.0 | 28.1 |
| SMN1 1x10³copy 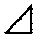 | 30.0 | 30.2 | 34.0 | 0.0 | 29.9 | 30.7 | 31.2 | 32.8 | 30.4 | 30.3 | 30.5 | 31.7 |
| *SMN1 1x10 ² copy* | ***33.3*** | ***33.5*** | ***37.4*** | ***0.0*** | ***33.5*** | ***33.9*** | **34.5** | ***36.1*** | ***33.6*** | ***33.5*** | ***33.8*** | ***34.8*** |
| SMN2 1x10⁵copy | 30.4 | 31.3 | 36.2 | 0.0 | 29.5 | 32.1 | 32.8 | 34.7 | 26.9 | 29.4 | 31.5 | 33.3 |
| *SMN2 1x10⁴ copy* | **33.5** | ***35.0*** | ***39.6*** | ***0.0*** | ***33.3*** | ***35.2*** | **36.6** | ***38.5*** | ***30.5*** | ***33.1*** | ***35.1*** | ***37.2*** |
| Cq Value 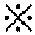 | **-0.2** | **-1.5** | **-2.2** | 0.0 | 0.2 | **-1.3** | **-2.1** | **-2.4** | 3.1 | 0.4 | **-1.4** | **-2.4** |

| | | | | | | | | | | | | |
|---|---|---|---|---|---|---|---|---|---|---|---|---|
| 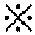 SMN1 1×10²copy Cq Value - SMN2 1×10⁴copy Cq Value | | | | | | | | | | | | |

### [Example 3] Examination of SMN2 Cross-Amplification Reaction of SMN1 Gene Quantification System with Coexisting Punched Piece of a Dried Blood Spot in a Filter Paper

### (a) Test Materials

### (a-1) SMN1 Gene Amplification Primers

As the primers for the *SMN*1 amplification, the following primers were synthesized by Sigma-Aldrich Co. LLC and used.
SMN1-specific forward primer; SEQ ID NO: 6 in Table 1 above
SMN1 common reverse primer; SEQ ID NO: 10 in Table 1 above

### (a-2) SMN1 Detection Probe

As the probe for observing the PCR amplification of *SMN1*, the following detection probe which was fluorescently labeled was synthesized and produced by Primetech Corporation and used.

### SMN1 Detection Probe

5'-(Quasar670)-GGAAGGTGCTCACATTCCTTAAATTAAGGA-(BHQ3)-3' (the nucleotide sequence part is SEQ ID NO: 23)

### (b) PCR Reagent

The composition of the PCR reagent is shown below.
PCR buffer (Ampdirect Plus; Shimadzu Corporation)
0.2 µM (final concentration) SMN1-specific forward primer
0.2 µM (final concentration) SMN1 common reverse primer
0.1 µM (final concentration) SMN1 detection probe
0.025 U/µL BIOTAQ HSDNA polymerase

### (c) PCR Reagents Containing Standards for Calibration Curve

A plasmid into which a partial sequence of SMN1 (SEQ ID NO: 11) was incorporated was added to the PCR reagent of (b) above, and PCR reagents containing the standards below (dilution series; STD1 to 4) were prepared. The values in the brackets are the copy numbers of the gene per one PCR reaction. The reagents were used in the solution state without adding to a dried blood spot in a filter paper.

### STD1 (SMN1 100,000 copy/reaction), STD2 (SMN1 10,000 copy/reaction), STD3 (SMN1 1000 copy/reaction), STD4 (SMN1 100 copy/reaction)

### (d) PCR Reagent for SMN2 Cross Test

A plasmid into which a partial sequence of SMN2 (SEQ ID NO: 12) was incorporated was added to the PCR reagent of (b) above at 50,000 copy/reaction, and a PCR reagent for *SMN2* cross test was thus prepared.

### (e) DNA-Free Dried Blood Spot in a Filter Paper

Blood obtained by mixing a human erythrocyte fraction from which leukocytes were removed and human plasma (EDTA) at a ratio of 6:4 in a volume of 40 µL was blotted on a filter paper for blood collection (Advantec) and directly dried, and the blood obtained was used as a DNA-free dried blood spot in a filter paper. This means that the dried blood spot in a filter paper did not contain *SMN1* and *SMN2.*

### (f) Conditions for PCR Reaction

(i) 95°C: 15 minutes
(ii) 95°C: 15 seconds, 63°C: 60 seconds (40 cycles)
(iii) 37°C: 5 minutes

### (g) Real-Time PCR Measurement

### (g-1) Measurement Method

The measurement was made by the following procedures.
(i) From the DNA-free dried blood spot in a filter paper, a circular punched piece with a diameter of 1.5 mm was taken using a puncher. As a control, a punched piece having the same size was taken from a filter paper for blood collection (Advantec) (empty punched piece).
(ii) To PCR reaction tubes (manufactured by Roche, Inc.: LightCycler8-TubeStrips, made of polypropylene), the punched pieces were introduced.
(iii) After adding 40 µL of the PCR reagent for *SMN2* cross test to the PCR reaction tubes, the tubes were sealed using the caps (included as a set with the tubes).
(iv) The PCR reagents containing the standards for a calibration curve in a volume of 40 µL were also dispensed to tubes (the tubes did not contain the punched pieces).
(v) Using a thermal cycler (LightCycler96; Roche, Inc.), real-time PCR measurement of *SMN1* and examination of the cross reaction to *SMN2* were conducted.

### (f-2) Measurement Results

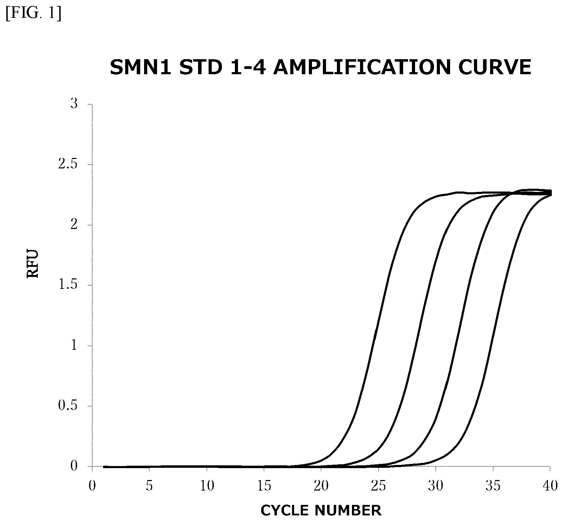

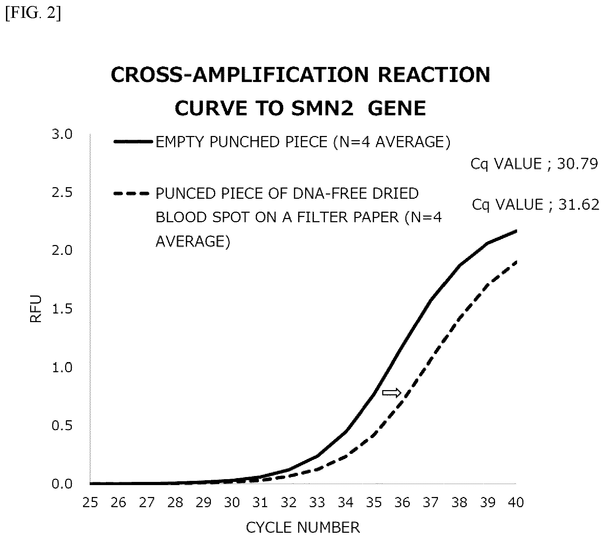

(i) The amplification reaction curves of the PCR reagents containing the standards for a calibration curve are shown in Fig. 1.
   It was observed that the amplification is excellent to SMN1 100 copy/reaction.
(ii) The parts of the amplification reaction curves of the PCR reagent for *SMN2* cross test at 25 to 40 cycles are shown in Fig. 2.

Interestingly, it was observed that the rising of the amplification delayed by Cq value of around 1 (the dotted line in the figure) when the punched piece of the DNA-free dried blood spot in a filter paper coexisted, as compared to the case where the empty punched piece (the punched piece containing no blood) coexisted (the solid line in the figure). This means that, under the conditions, the cross reaction to *SMN2*, in terms of the copy number, decreased by about 50%. That is, it was found that, when a primer designed in a manner that the reactivity to the *SMN2* gene is less than 1% of that to the *SMN1* gene in the invention is applied to a case where a dried blood spot in a filter paper is used as an analyte, the reactivity to the *SMN2* gene weakens. This means that it was found that, when PCR reaction is performed using the primer of the invention, the cross reaction to the *SMN2* gene is reduced more in the presence of a punched piece of a dried blood spot in a filter paper in the reaction system.

Therefore, the invention leads to a reduction in the risk of overlooking a SMA patient having homozygous deletion of *SMN1* (false negative) in newborn screening test.

Here, when a test was conducted in the same manner also regarding *SMN*1, inhibition of the amplification by a dried blood spot in a filter paper was not observed (the results are not shown in the figures). Therefore, it was found that the phenomenon of the delayed rising of the amplification due to the coexistence of a dried blood spot in a filter paper is specific to the amplification reaction of the *SMN2* gene. Thus, in other words, the invention of the present application is a method for weakening the reactivity to the *SMN2* gene using a specific primer in a method for detecting the *SMN1* gene in a dried blood spot in a filter paper by real-time PCR.

### INDUSTRIAL APPLICABILITY

According to the invention, the provision of a method for specifically detecting or quantifying the *SMN1* gene in a dried blood spot in a filter paper by real-time PCR has been enabled using the specific primer of the invention. Therefore, a rapid detection/quantification system for the *SMN1* gene which can be applied to newborn screening in which the symptoms tend to become severe and in which the treatment is desirably started at an early stage can be provided.

## Claims

1. A method for detecting the *SMN1* gene in a dried blood spot in a filter paper by real-time PCR, including the steps of (A) to (D) below:
(A) a step of adding the dried blood spot in a filter paper to a PCR reaction tube;
(B) a step of adding a PCR reagent to the PCR reaction tube, wherein the PCR reagent contains at least a primer designed in a manner that the reactivity to the *SMN2* gene is less than 1% of that to the *SMN1* gene, a polymerase, dNTPs and an intercalator or a fluorescently labeled probe;
(C) a step of performing PCR reaction in the tube containing the PCR reagent and the dried blood spot in a filter paper; and
(D) a step of sequentially and optically detecting a target nucleic acid in the *SMN1* gene amplified by the PCR reaction,
wherein the primer designed in a manner that the reactivity to the *SMN2* gene is less than 1% of that to the *SMN1* gene is one or more primers selected from the group consisting of the forward primer of (1) and the reverse primer of (2) below:
(1) a forward primer having any of the nucleotide sequences of SEQ ID NOs: 1 to 9; and
(2) a reverse primer having any of the nucleotide sequences of SEQ ID NOs: 16 to 21.

2. The detection method according to claim 1, wherein the dried blood spot in a filter paper is a circular punched piece with a diameter of 1.2 mm to 2.0 mm or a punched piece containing whole blood in an amount of 0.95 v/v% to 6.6 v/v% based on the total amount of the reaction solution.

3. Use of a primer for specifically detecting the *SMN1* gene in a dried blood spot in a filter paper as a sample which is one or more primers selected from the group consisting of the forward primer of (1) and the reverse primer of (2) below:
(1) a forward primer having any of the nucleotide sequences of SEQ ID NOs: 1 to 9; and
(2) a reverse primer having any of the nucleotide sequences of SEQ ID NOs: 16 to 21.

4. A method for weakening the reactivity to the *SMN2* gene in a method for detecting the *SMN1* gene in a dried blood spot in a filter paper as a sample by real-time PCR which uses one or more primers selected from the group consisting of the forward primer of (1) and the reverse primer of (2) below:
(1) a forward primer having any of the nucleotide sequences of SEQ ID NOs: 1 to 9; and
(2) a reverse primer having any of the nucleotide sequences of SEQ ID NOs: 16 to 21.

## Patentansprüche

1. Verfahren zum Nachweis des *SMN1-Gens* in einem getrockneten Blutfleck in einem Filterpapier anhand von Echtzeit-PCR (real-time PCR), umfassend die folgenden Schritte (A) bis (D):
(A) einen Schritt der Zugabe des getrockneten Blutflecks in einem Filterpapier in ein PCR-Reaktionsgefäß;
(B) einen Schritt der Zugabe eines PCR-Reagens zu dem PCR-Reaktionsgefäß, wobei das PCR-Reagens mindestens einen Primer, der so konzipiert ist, dass die Reaktivität gegenüber dem *SMN2*-Gen weniger als 1% derjenigen gegenüber dem *SMN1-Gen* beträgt, eine Polymerase, dNTPs und einen Interkalator oder eine fluoreszenzmarkierte Sonde enthält;
(C) einen Schritt der Durchführung einer PCR-Reaktion in dem Gefäß, das das PCR-Reagenz und den getrockneten Blutfleck in einem Filterpapier enthält; und
(D) einen Schritt des sequentiellen und optischen Nachweises einer Zielnukleinsäure in dem anhand der PCR-Reaktion amplifizierten *SMN1-Gen,* wobei der Primer, der so konzipiert ist, dass die Reaktivität gegenüber dem SMN2-Gen weniger als 1 % derjenigen gegenüber dem *SMN1-*Gen beträgt, ein oder mehrere Primer ist, die aus der Gruppe ausgewählt sind, die aus dem folgenden Vorwärtsprimer von (1) und dem folgenden Rückwärtsprimer von (2) besteht:
(1) ein Vorwärtsprimer mit einer der Nukleotidsequenzen gemäß SEQ ID NOs: 1 bis 9; und
(2) ein Rückwärtsprimer mit einer der Nukleotidsequenzen gemäß SEQ ID NOs: 16 bis 21.

2. Nachweisverfahren nach Anspruch 1, wobei der getrocknete Blutfleck in einem Filterpapier ein kreisförmiges ausgestanztes Stück mit einem Durchmesser von 1,2 mm bis 2,0 mm oder ein ausgestanztes Stück ist, das Vollblut in einer Menge von 0,95 v/v% bis 6,6 v/v%, bezogen auf die Gesamtmenge der Reaktionslösung, enthält.

3. Verwendung eines Primers zum spezifischen Nachweis des *SMN1-Gens* in einem getrockneten Blutfleck in einem Filterpapier als Probe, wobei es sich um einen oder mehrere Primer handelt, die aus der Gruppe ausgewählt sind, die aus dem folgenden Vorwärtsprimer von (1) und dem folgenden Rückwärtsprimer von (2) besteht:
(1) ein Vorwärtsprimer mit einer der Nukleotidsequenzen gemäß SEQ ID NOs: 1 bis 9; und
(2) ein Rückwärtsprimer mit einer der Nukleotidsequenzen gemäß SEQ ID NOs: 16 bis 21.

4. Verfahren zur Abschwächung der Reaktivität auf das *SMN2-*Gen in einem Verfahren zum Nachweis des *SMN1-Gens* in einem getrockneten Blutfleck in einem Filterpapier als Probe anhand von Echtzeit-PCR, das einen oder mehrere Primer verwendet, die aus der Gruppe ausgewählt sind, die aus dem folgenden Vorwärtsprimer von (1) und dem folgenden Rückwärtsprimer von (2) besteht:
(1) ein Vorwärtsprimer mit einer der Nukleotidsequenzen gemäß SEQ ID NOs: 1 bis 9; und
(2) ein Rückwärtsprimer mit einer der Nukleotidsequenzen gemäß SEQ ID NOs: 16 bis 21.

## Revendications

1. Procédé de détection du gène *SMN1* dans une tache de sang séché dans un papier-filtre par PCR en temps réel, incluant les étapes de (A) à (D) ci-dessous :
(A) une étape d'ajout de la tache de sang séché dans un papier-filtre à un tube de réaction PCR ;
(B) une étape d'ajout d'un réactif PCR au tube de réaction PCR, dans lequel le réactif PCR contient au moins une amorce conçue de manière à ce que la réactivité au gène *SMN2* soit inférieure à 1 % de celle au gène *SMN1,* à une polymérase, à des dNTP et à un intercalant ou à une sonde de marquage fluorescent ;
(C) une étape de réalisation d'une réaction PCR dans le tube contenant le réactif PCR et la tache de sang séché dans un papier-filtre ; et
(D) une étape de détection séquentielle et optique d'un acide nucléique cible dans le gène *SMN1* amplifié par la réaction PCR,
dans lequel l'amorce conçue de manière à ce que la réactivité au gène *SMN2* soit inférieure à 1 % de celle au gène *SMN1* est une ou plusieurs amorces sélectionnées parmi le groupe consistant en l'amorce avant de (1) et l'amorce inverse de (2) ci-dessous :
(1) une amorce avant présentant l'une quelconque des séquences de nucléotide des SEQ ID NO : 1 à 9 ; et
(2) une amorce inverse présentant l'une quelconque des séquences de nucléotide des SEQ ID NO : 16 à 21.

2. Procédé de détection selon la revendication 1, dans lequel la tache de sang séché dans un papier-filtre est une pièce perforée circulaire d'un diamètre de 1,2 mm à 2,0 mm ou une pièce perforée contenant du sang total en une quantité de 0,95 % v/v à 6,6 % v/v sur la base de la quantité totale de la solution de réaction.

3. Utilisation d'une amorce pour détecter spécifiquement le gène *SMN1* dans une tache de sang séché dans un papier-filtre en tant qu'échantillon qui est une ou plusieurs amorces sélectionnées parmi le groupe consistant en l'amorce avant de (1) et l'amorce inverse de (2) ci-dessous :
(1) une amorce avant présentant l'une quelconque des séquences de nucléotide des SEQ ID NO : 1 à 9 ; et
(2) une amorce inverse présentant l'une quelconque des séquences de nucléotide des SEQ ID NO : 16 à 21.

4. Procédé d'affaiblissement de la réactivité au gène *SMN2* dans un procédé de détection du gène *SMN1* dans une tache de sang séché dans un papier-filtre en tant qu'échantillon par PCR en temps réel qui utilise une ou plusieurs amorces sélectionnées parmi le groupe consistant en l'amorce avant de (1) et l'amorce inverse de (2) ci-dessous :
(1) une amorce avant présentant l'une quelconque des séquences de nucléotide des SEQ ID NO : 1 à 9 ; et
(2) une amorce inverse présentant l'une quelconque des séquences de nucléotide des SEQ ID NO : 16 à 21.
